(19) Europäisches Patentamt
European Patent Office
Office européen des brevets

(11) **EP 3 449 813 A1**

(12) **EUROPEAN PATENT APPLICATION**

(43) Date of publication:
**06.03.2019 Bulletin 2019/10**

(51) Int Cl.:
**A61B 5/00** (2006.01) **A61B 5/024** (2006.01)
**A61B 5/1455** (2006.01)

(21) Application number: **17189314.2**

(22) Date of filing: **05.09.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **PRESURA, Cristian Nicolae**
**5656 AE Eindhoven (NL)**

• **HUIJBREGTS, Laurentia Johanna**
**5656 AE Eindhoven (NL)**
• **HILGERS, Achim**
**5656 AE Eindhoven (NL)**
• **AARTS, Ronaldus Maria**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **PHYSIOLOGICAL CONTACT SENSOR WITH NO EMBEDDED LIGHT SOURCE**

(57) A portable device for determining physiological parameters of a user using ambient light is provided. A first and a second photo detector are used to determine a first signal comprising a first ambient light component and a second signal comprising a second ambient light component along with a modified ambient light component, respectively. The first signal is used to correct the second signal in order to extract the modified ambient light component and determine the physiological parameter on the basis of said modified ambient light component.

**FIG. 1**

**Description**

FIELD OF THE INVENTION

The present invention relates to a portable device for determining a physiological parameter of a user using ambient light and a corresponding method. More particularly, the invention relates to a device wearable by a user during outdoor activity wherein the ambient light is used for measurements of the physiological parameter.

BACKGROUND OF THE INVENTION

Due to the growing health awareness in nowadays society, many products for determining physiological parameters have been developed in the past years. Many of these products relate to devices that are portable and/or wearable and should be carried or worn by the users the entire time, 24 hours a day and seven days a week.

The determination of physiological parameters is usually performed using a combination of an optical sensor and an artificial light source integrated into these devices. The artificial light source provides light to the skin of the user which is reflected and/or transmitted from the skin and detected by the optical sensor, usually including a photo detector. To that end, typical physiological parameters that are measured relate to information monitored during the cardiac cycle, such as the heart rate or oxygen saturation in the blood. These physiological parameters may be measured using optical heart rate monitors, such as photoplethysmography (PPG) sensors, that may be attached to different parts of the body, such as the wrist, the earlobe or the finger of the user.

A major issue of devices including optical sensors is their susceptibility to motion artifacts, especially in the case of fast or striding movement of the user, as typically occurring during physical activity. Several approaches have been taken to mitigate this problem.

In that respect, WO 2013/038296 suggests to measure the motion of a body part of a person to be monitored in parallel to the heart rate measurement and to estimate a corrected heart rate if the quality of the heart rate measurement is below a certain threshold.

Another problem of devices employing optical sensor measurements to determine physiological parameters is their high power consumption which necessitates regular recharging of the devices' batteries. Hereby, a significant amount of the power is consumed by the artificial light sources implemented in the devices.

In order to reduce power consumption for the devices, some approaches aim at replacing the light produced by these artificial light sources with ambient light for the optical sensor measurements. This avoids the necessity of an additional light source which, in turn, reduces the device's power consumption and weight, thereby improving user comfort.

However, using ambient light for optical sensors to measure physiological parameters is a delicate issue, as ambient light is subject to strong fluctuation. This fluctuation is influenced by a multitude of different factors. On the one hand, fluctuation of the ambient light may be caused by movement of the user. The user may, for example, move his or her arm closer to the body. The body would then shield a particular amount of ambient light, thereby inducing a fluctuation in the ambient light.

Further, the fluctuation may also be inherent to the ambient light. For example, the ambient light may be brighter in the middle of the day than in the late evening. Finally, a fluctuation may also be caused by the user moving from one place to another, for example when a user moves from an open space of bright ambient light into a secluded place with only little ambient light. Thus, optical sensor measurements of portable devices using ambient light as the sole light source are, up to this day very error-prone.

SUMMARY OF THE INVENTION

It is an object of the present invention to provide an improved device and method for determining a physiological parameter using optical sensor measurements which provides increased user comfort.

It is a further objection of the present invention to provide an improved portable device for determining physiological parameter for which power consumption may be reduced. More particularly, it is an object of the invention to provide a portable device for determining a physiological parameter which employs ambient light as the primary light source and which enables a reliable optical sensor measurement of physiological parameters.

The object of the present invention is solved by a portable device according to the invention and a corresponding method for determining a physiological parameter of a user.

According to a first aspect of the invention, a portable device for determining a physiological parameter of a user using ambient light is provided, the portable device comprising a first photo detector adapted to obtain a first signal comprising a first ambient light component and a second photo detector adapted to obtain a second signal comprising a combination of a second ambient light component and a modified ambient light component. The device further comprises a processing unit adapted to determine the physiological parameter from the first signal and the second signal.

In accordance with the invention, a portable device may in particular be a wearable device that may be worn by a user. The user may wear the device around the wrist or attach it to his or her ear lope or fingertip. Further positions for the device may be imagined, as long as they allow ambient light to be incident on the skin.

A physiological parameter may be any parameter that is related to the physiological status of the user.

Specific examples for the physiological parameter may be the heart rate or the oxygen saturation of the blood. The determination of said physiological parameter may follow known principles, such as photoplethysmography (PPG) using a pulse oximeter. Hereby, the light having traveled through the skin is measured by a photo detector. On its way through the skin, the light encounters blood vessels. Due to the heart beating during the cardiac cycle, the blood moving through these blood vessels varies. Thus, the light travelling through the blood vessels is absorbed differently, depending on the variation of the blood in the blood vessels. This varying absorption causes a respective signal variation on the photo detector. The signal on the photo detector may then be used to derive the physiological parameters.

For the special case of PPG, the high frequency part s(t) of the signal on the photo detector comprises a pulsating component which may be approximated by

$$s(t) = a(t)\sin(2\pi f * t + \alpha).$$

Hereby, f is the frequency of the heartbeat and $a(t)$ is a time-dependent component accounting for motion artifacts and fluctuation in the light.

In the context of the present invention, the first photo detector and the second photo detector may be embodied as photo diodes, photo transistors or the like.

Hereby, the first photo detector is used to properly estimate the time-dependent component $a(t)$. The first photo detector is used to obtain a first signal that contains a first ambient light component.

In the context of this invention, the term first ambient light component refers to a component of the first signal obtained by the first photo detector which is indicative of ambient light that has not interacted with the skin and/or blood vessels and, thus, not altered prior to being measured.

This may be achieved by arranging the first photo detector such that the ambient light may be directly incident on the first photo detector. Alternatively, the first photo detector may also be arranged such that the ambient light is not directly incident thereon, but has been reflected elsewhere prior to reaching the first photo detector. Hereby, it may be necessary to filter the light incident on the first photo detector, such that only the part of the light that constitutes the first ambient light component may pass through the filter whereas the remaining components are filtered out.

Accordingly, the first signal is proportional to $a(t)$:

$$m(t) \sim a(t).$$

To that end, the second photo detector is provided to obtain a second signal that comprises a second ambient light component and a modified ambient light component.

In this context, the term second ambient light component refers to a component of the second signal which is indicative of ambient light that has not been altered by encountering skin and/or blood vessels. The term modified ambient light component refers to a component of the second signal that is indicative of ambient light that has been modified by interacting with the skin and/or the blood vessels therein.

In other words, the second signal obtained by the second photo detector is indicative of a mixture of ambient light that has not been altered by interaction with the skin and/or the blood vessels and ambient light that has been modified due to interaction therewith.

In accordance with the invention, the first ambient light component of the first signal may be used as an indication of the changes in the ambient light due to fluctuations, e.g. because of movement or environmental factors (clouds, shades or the like). The second signal likewise contains a component affected by fluctuations of the ambient light, namely the second ambient light component, but further comprises the modified ambient light component which is indicative of the pulsating component that may be used to determine the physiological parameters. Since the first photo detector and the second photo detector thus measure the ambient light in parallel, the second signal obtained by the second photo detector may be corrected for fluctuations caused by movement, shades or the like using the first signal obtained by the first photo detector. This correction may be approximated as follow:

$$s_{corr}(t) = \frac{s(t)}{m(t)} = C * \sin(2\pi f * t + \alpha).$$

Here, C is the correction factor used to correct for fluctuations caused in the ambient light due to motion of the skin or the body part or due to other factors.

According to one embodiment, the first photo detector is arranged on a first side of the user's skin and the second photo detector is arranged on a second side of the user's skin opposite the first side along a propagation direction of the ambient light through the user's skin.

In a first embodiment, the device is implemented such that, when the device is worn by a user, the first photo detector is placed on one side of a user's skin in a manner that the ambient light may be directly incident on it. This may be achieved by placing the photo detector such that the sensor faces away from the user's skin, i. e. having the sensor face the incident light. The device is further implemented such that the second photo detector is placed on another side of the user's skin opposite to the first photo detector along the propagation direction of the light through the user's skin. Hereby, the second photo detector could be arranged inside the device in a manner that the second photo detector is largely shielded from direct ambient light, such that the modified ambient light component may be sufficiently detected in the second signal. This may for example be achieved by surrounding the second photo detector with an opaque material.

In this embodiment, the device may for example be implemented as an ear piece that may be attached to the ear lobe of a user. The first photo detector may be arranged on the device such that it is placed on the front side of the auricle, when the device is worn. The second photo detector may be arranged on the device such that it is placed on the back side of the auricle in that case.

In this embodiment, the measurements of the first and second signals may be performed in transmission. The first signal obtained by the first photo detector comprises the first ambient light component, which in this case is caused by direct light incident on the first photo detector. This light has not encountered the skin and, thus, directly provides a measurement of the fluctuations in the ambient light due to movement and environmental factors.

The second signal obtained by the second photo detector comprises the second ambient light component and the modified ambient light component. The second ambient light component hereby indicates changes in the second signal that are caused by fluctuations in the ambient light due to movement or environmental factors and the modified ambient light component has been affected by interaction with the skin and/or the blood vessels. Thus, the modified ambient light component is indicative of the pulsating component representing changes in blood flow of the user. By measuring the first signal and using the measurements to approximate the fluctuation in the ambient light, the second ambient light component and modified ambient light component in the second signal may be distinguished from one another, thereby correcting the measurement of the second photo detector. This reduces the probability of erroneous measurements of the physiological parameter due to fluctuations in the ambient light itself.

According to a further embodiment, the first photo detector and the second photo detector are arranged on a first side of the user's skin and the first photo detector comprises a first filter adapted such that the first ambient light component may pass the first filter and the second photo detector comprises a second filter adapted such that the second ambient light component and the modified ambient light component may pass the second filter.

The first filter may hereby comprise a red and/or infrared filter and the second filter may comprise a green filter.

The device may be implemented such that the first photo detector and the second photo detector are arranged on the same side of the user's skin. As an example, the first photo detector and the second photo detector may be arranged next to each other on a device having the shape of a wrist band. In this case, the first photo detector and the second photo detector should not be placed too far from the side of the device, such that the ambient light coming from the skin may reach the first and the second photo detector.

In this arrangement, the light incident on the first photo detector as well as the light incident on the second photo detector has at least partially been altered by encountering the skin and/or blood vessels. Accordingly, in principle, the first signal and the second signal should comprise the modified light component. In order to avoid said modified light component in the first signal a respective first filter is used. The first filter is hereby arranged in front of the first photo detector along the propagation direction of the ambient light. The ambient light therefore has to pass the first filter prior to being incident on the first photo detector. Hereby, the part of the ambient light that has been modified by interaction with the skin and/or blood vessels is filtered from the ambient light. Thus, the first signal obtained by the first photo detector comprises only the first ambient light component.

Further, a respective second filter is arranged in front of the second photo detector along the propagation direction of the ambient light. The second filter is selected such that the light that has been modified by encountering skin and/or bloods vessels may pass the filter and be incident on the second photo detector. Thus, the second signal comprises the second ambient light component and the modified ambient light component.

In the context of the application, the terms first filter and second filter may be understood to describe a single first filter and a single second filter, each for performing a single filter operation. However, the first filter and the second filter may also each refer to a combination of multiple filters for multiple filter operations.

In that respect, the first filter may particularly comprise a red or infrared filter, which allows the red or infrared spectrum of the ambient light to pass the filter and to be incident on the first photo detector. The second filter may particularly comprise a green filter, which allows the green spectrum of the ambient light to pass the filter and to be incident on the second photo detector. The absorption of ambient light by the blood vessels is much larger in the green spectrum than in the red or infrared spectrum of the ambient light. Accordingly, the second photo detector detects the spectral regime of the ambient light in which the modified ambient light component is highly pronounced. As a result, the second signal obtained by the second photo detector comprises a combination of the second (unmodified) ambient light component and the modified ambient light component. Since the absorption in the red spectrum is accordingly very small, the first photo detector detects the spectral regime of the ambient light in which the modified light component is less pronounced. Thus, the first signal obtained by the first photo detector mainly comprises the first (unmodified) ambient light component.

The measurements performed by means of this embodiment may be done in reflection, in transmission or by a combination of both.

The first and second photo detector may perform the measurements in reflection. The ambient light incident on the skin is hereby reflected from the skin and the blood vessels therein and reaches the first and second photo detector through the first and second filter, respectively.

Since the ambient light is reflected from the skin, the propagation path of the light through the skin is rather short. This makes measurements in reflection particularly well-suited in case of devices which are worn on larger body parts, such as the wrist or the ankle. A particularly good signal may hereby be achieved if the side of the skin on which the first and second photo detector are arranged directly faces the ambient light source (e.g. the sun).

The first and second photo detector may further perform the measurements in transmission. Hereby, the light detected by the first and second photo detector is transmitted from one side of the skin to another side of the skin along a propagation direction of the ambient light. In this case, the propagation path of the light through the skin is longer, since the light has to pass the skin. Measurements in transmission are particularly well-suited in case of devices which are worn at smaller, thinner body parts, such as the ear lobe.

Finally, the first and second photo detector may also perform measurements using light from both, transmission and reflection. In this case, modified ambient light component has a reflected modified ambient light component and a transmitted modified light component, i.e. the light is modified by being reflected from the skin and by having travelled through the skin along a propagation path. In reality, in most measurements, the modified ambient light component will comprise a transmitted and a reflected modified ambient light component.

According to a modification, the device further comprises an indicator. The first photo detector comprises a first ultraviolet filter and the first signal obtained by the first photo detector comprises a first ultraviolet light component. Further, the second photo detector comprises a second ultraviolet filter and the second signal obtained by the second photo detector comprises a second ultraviolet light component. The processing unit is further adapted to provide a first indication to the indicator upon determining that a value of the first ultraviolet light component is above a pre-determined first ultraviolet light threshold and to provide a second indication to the indicator upon determining that a value of the second ultraviolet light component is above a pre-determined second ultraviolet light threshold. According to this modification, the first ultraviolet filter and the second ultraviolet filters are arranged in front of the first photo detector and the second photo detector, respectively. The first and second ultraviolet filters allow a first and second ultraviolet component to be incident on the first and second photo detector, respectively. Thus, the first and second photo detector may each be used to detect ultraviolet radiation.

In the context of the invention, the first ultraviolet light component is indicative of the ultraviolet radiation in the ambient light. The first signal obtained by the first photo detector comprises the first ultraviolet light component and the processing unit may derive a first intensity value for the first ultraviolet light component from the first signal. The processing unit may then compare the first intensity value of the first ultraviolet light component to a first ultraviolet light threshold that has been pre-determined in the processing unit. In this context, the term pre-determined means that the first threshold may have been previously selected from a number of stored threshold values by a user or pre-set by a manufacturer.

In case the processing unit determines that the first intensity value for the first ultraviolet light component is above the first ultraviolet light threshold, the processing unit provides an indication to the indicator that the threshold has been exceeded. The indicator may then instruct the user to take particular measures. As an example, the first intensity value for the first ultraviolet component exceeds the first ultraviolet light threshold because of a sudden sun burst. The processing unit may, in response to determining that the threshold has been exceeded, prompt the indicator to instruct the user to exit the sun immediately. The user may hereby be instructed to move into a shadow or into a building, to use sunscreen or the like. Alternatively or additionally, the indicator may also simply indicate the sun burst and the user may decide how to react by him- or herself.

Likewise, the second ultraviolet light component may be comprised in the second signal. Hereby, the second ultraviolet light component may particularly be indicative of the ultraviolet radiation in the ambient light that has at least partly encountered and interacted with the skin and/or blood vessels of a user. The second signal obtained by the second photo detector comprises the second ultraviolet light component and the processing unit receiving the second signal can derive a second intensity value for the second ultraviolet light component from the second signal and compare the second intensity value to a second ultraviolet light threshold that has been pre-determined in the processing unit. In this case also, the pre-determination may have been performed by the user selecting a certain threshold or by the manufacturer setting a threshold value. Other methods for pre-determining the threshold values may also be used.

In case the processing unit determines that the second intensity value for the second ultraviolet light component is above the second ultraviolet light threshold, the processing unit provides an indication to the indicator that the second threshold has been exceeded, upon which the indicator instructs the user accordingly. Particularly, the indicator may indicate to the user that the second intensity value for the second ultraviolet light component exceeds the second ultraviolet light threshold because of a lack of sunscreen applied to the skin of the user. The indicator may then instruct the user to apply more sunscreen. Alternatively or additionally, the indicator may also simply inform the user that the sunscreen is insufficient and the user may decide whether he or she will apply more sunscreen or move out of the sun or take other measures.

Optionally, the processing unit may use the first signal comprising the first ultraviolet light component to estimate the intensity value for the second ultraviolet component that has encountered the user's skin, i.e. the processing unit may use the first signal to correct the second signal with respect to the first ultraviolet component as well.

The processing unit may compare the values for the first and second ultraviolet light components to the first and second ultraviolet light thresholds directly after activation of the device. Further, the processing unit may periodically repeat the comparison during the entire operation time of the device. Optionally, the device may offer an input means to the user, in which the user may select to activate or deactivate the comparison of the first and second ultraviolet light components to their respective pre-determined thresholds.

The indicator may provide the instruction to the user in the form of a text message shown on a display of the device. Alternatively or additionally, the instruction may also comprise a signal, such as a light signal or a vibration signal. The indicator may also provide an auditory signal via a speaker in which the instruction is provided in spoken form. Likewise, the auditory signal may be a short signal tone.

According to an embodiment, the device further comprises a light guide for guiding the ambient light to a user's skin, the light guide comprising a first outlet and a second outlet for directing the ambient light to a first side of the user's skin.

According to a further embodiment, the first photo detector is arranged adjacent to the first outlet and the second photo detector is arranged adjacent to the second outlet on the first side of the user's skin. In an even further embodiment, the first outlet comprises a first filter and the second outlet comprises a second filter.

The ambient light may be guided to the user's skin using a light guide such as a fiber. The light guide may hereby be arranged to allow the ambient light to enter from the top and the sides. The ambient light is then guided to the skin by the light guide. The light guided by the light guides then enters the skin and interacts with the skin and the blood vessels.

The first and second photo detectors are hereby arranged such that they may obtain the first and second signal respectively from the light guided to the skin. More particularly, the first and second photo detector may be arranged in the vicinity of the first and second outlet of the light guide on the same side of the user's skin. This arrangement may be used for example for devices worn around the wrist or ankle. In this case, the first and second photo detector may in particular obtain the signal from a reflected ambient light component, wherein the ambient light has been reflected from the skin or from a combination of a reflected and transmitted ambient light component.

It is also possible to arrange the first photo detector directly below the first outlet of the light guide and the second photo detector adjacent to the second outlet of the light guide. By means of this arrangement, the light guided through the first outlet is directly incident on the first photo detector. Thus, the first photo detector may obtain a first signal having a clear first ambient light component. The second photo detector may hereby obtain the second signal as described above.

Alternatively, the first and second photo detector may be arranged on the side opposite the first and second outlet of the light guide along a propagation direction of the light through the user's skin. This arrangement may be used for example in case of an ear clip or a finger clip. In this case, the first and second photo detector may in particular obtain the signal from a transmitted ambient light component.

Furthermore, the first and second photo detectors may be arranged on opposite sides of the user's skin along the propagation direction of the ambient light. As an example, the first photo detector may be arranged on the same side as the first and second outlet of the light guide, in particular in the vicinity of one or directly below one of the first or second outlet. The second photo detector may be arranged on the side of the user's skin that is opposite the side on which the first and second outlet of the light guide is arranged. In this case, the first photo detector may obtain the first signal comprising the first ambient light component in reflection or directly and the second photo detector may obtain the second signal comprising the second ambient light component and the modified ambient light component in transmission.

The first and second photo detectors are hereby used to obtain the first and second signal as described herein above. By means of this arrangement, it is possible to focus the ambient light which may lead to a better signal quality on the photo detectors.

Optionally, a first optical light guide filter and a second optical light guide filter are arranged at the outlets of the light guide. The light guided through the light guide thus has to pass the first and second light guide filters prior to entering the skin of the user. These first and second light guide filters may optionally be selected such that they let only certain wavelengths pass. Accordingly, the first and second light guide filters may be used to prevent measurements in a particular spectral regime of the ambient light, in particular to limit the spectral regime to the wavelengths that are actually required for the measurement. Alternatively or additionally, the first and second light guide filters may be used to prevent saturation effects on the first and second photo detectors. These saturation effects may in particular occur when there are high intensity ambient light sources close to the user.

The first and second light guide filters may be separate components that have to be attached to the light guide at appropriate positions for filtering. Alternatively or additionally, the first and second light guide filters may also be provided as part of the light guide, for example by means of a coating of the light

In the context of this embodiment, different filter configurations may be used. For filtering the ambient (sun) light and for irradiation of defined areas of the user's skin using a limited spectral regime, i.e. only a limited wavelength such as a certain color, optical filters may particularly be used. As an example, high-pass filters, low-pass filters or band pass filters are mentioned. Likewise, interference filters may be used. The filters may also be used to block parts of the spectral regime, e.g. by means of a band stop filter or to absorb the whole spectrum. These configurations may particularly be used to limit the region at which the light should irradiate the skin.

To this end, the suitable filter configurations and materials for producing these filters are known in the art, e.g. from Schott.

In a further embodiment, the device comprises an accelerometer arranged in close proximity to the second photo detector. The accelerometer is adapted to output a third signal indicative of the motion of a user's skin and the processing unit is adapted to determine the physiological parameter from the first signal, the second signal and the third signal.

According to this embodiment, the device may further comprise an accelerometer which may particularly be used to eliminate motion artifacts occurring in the ambient light that have been caused by movement of the user, in particular by movement of the user's skin. The accelerometer is hereby attached to the skin via the device and is used to determine the motion of the user and/or the user's skin. On the basis of this motion, the accelerometer can derive position changes of the device relative to the ambient light source. The accelerometer may then output a corresponding third signal that indicates said motion of the user and/or the user's skin. This third signal is provided to the processing unit. The processing unit then uses the third signal to determine which motion has been performed by the user. By means of this determination, the processing unit is in particular enabled to determine whether a certain fluctuation in the ambient light has been caused by the motion of the user. The processing unit may then correct the pulsating component of the second signal using this information. Thus, by adding an accelerometer to the device, the reliability of the measurement of the physiological parameter may further be improved.

In yet another embodiment, the device further comprises a light source and a controller unit. The processing unit is adapted to determine whether an intensity value of the ambient light is below or above a pre-defined threshold and the processing unit is adapted to cause the controller unit to activate the light source when it is determined that the intensity value of the ambient light is below the pre-defined threshold.

According to another embodiment, the light source comprises one or more light emitting detectors and the controller is further adapted to control the light emitting detectors to provide a change in intensity.

In some cases, the device may be used in an environment where no or only little ambient light is present. In order to ensure that the device may still be used to determine physiological parameters, the device is provided with an additional artificial light source. In this case, the processing unit determines an intensity value for the ambient light from the first and/or second signal and compares this intensity value to a pre-defined threshold value. In case it is determined that the intensity value for the ambient light is below said pre-defined threshold value, the processing unit sends an activation signal to the controller. The controller then activates the additional, artificial light source. Additionally, the processing unit may also send a signal to the indicator, in order to inform the user that the additional light source is now employed. The pre-defined threshold value may hereby particularly be pre-set during the manufacturing process in accordance with the sensitivity of the first and second photo detector and the arrangement of said photo detectors. It should be selected such that the additional light source is activated when the intensity of the ambient light is too small to enable a reliable determination of the physiological parameters.

The controller is hereby arranged to adjust the intensity of the light source in accordance with the intensity value determined for the ambient light such that a sufficient intensity of the light is achieved such that a reliable determination of the physiological parameters may be performed. For example, the controller may have access to data about certain intensity values for the ambient light and adjust the intensity of the light source such that the combination of the ambient light and the artificial light by the light source is above the intensity threshold necessary to obtain signals of a certain signal strength and signal-to-noise-ratio.

The light source may hereby particularly comprise one or more light emitting detectors (LEDs) that are controlled by the controller. In case of multiple light emitting detectors, the control may be performed selectively, such that groups of light emitting detectors are controlled together or each light emitting detector is controlled individually. In particular, a selective or individual on- or off-switching of the light emitting detectors may be used to change the intensity of the light output of the light source. Alternatively or additionally, the intensity of each of the light emitting detectors may also be changed by the controller, e.g. by the controller dimming the light emitting detectors.

In yet another embodiment, the device comprises a first attachment portion adapted for attaching the first photo detector, the first attachment portion comprising a transparent material and a second attachment portion adapted for attaching the second photo detector, the second attachment portion comprising an opaque material.

According to a further embodiment, the device is a wearable device, such as an ear clip, a finger clip or a wrist-worn device.

According to the invention, the first photo detector should detect a first signal indicative of the ambient light. Thus, it is beneficial if the first photo detector receives as much ambient light as possible. This may particularly be achieved by providing the device with a first attachment portion for the first photo detector that is made from a transparent material, such as a transparent plastic. The ambient light may then travel directly through the attachment portion to the first photo detector and the first photo detector will obtain a good first signal comprising the first ambient light component.

Further, the second photo detector should be shielded from direct ambient light as good as possible in order to pronounce the modified ambient light component. This may be achieved by providing the device with an opaque second attachment portion to which the second photo detector may be attached. As an example, the attachment portion for the second photo detector may be a wrist band or a finger or ear clip that has be manufactured from a fabric or plastic. The fabric or plastic may particularly be of a dark color, e.g. a dark blue or green or black.

Hereby, the first and second attachment portion may be provided on the same side of the skin or on opposite sides depending on how the first and second photo detectors shall be arranged. It is also possible to attach both, the first and the second photo detector to the second attachment portion. In this case, the first attachment portion is merely used as a counterpart for attaching the device to the user.

In a second aspect, the invention relates to a method for determining a physiological parameter of a user using a portable device using ambient light comprising obtaining a first signal comprising a first ambient light component by a first photo detector, obtaining a second signal comprising a combination of a second ambient light component and a modified ambient light component a second photo detector and determining the physiological parameter from the first signal and the second signal using a processing unit.

It shall be understood that the device of claim 1 and the method of claim 14 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 schematically shows an arrangement of the first photo detector and the second photo detector according to a first embodiment.
Fig. 2 schematically shows an arrangement of the first photo detector and the second photo detector according to a second embodiment.
Fig. 3 shows an absorption spectrum for deoxygenated and oxygenated blood.
Fig. 4 schematically shows an arrangement of the first photo detector, the second photo detector and an accelerometer according to a modification.
Fig. 5 shows a device for determining a physiological parameter of a user using ambient light according to a first embodiment.
Fig. 6 shows a device for determining a physiological parameter of a user using ambient light according to a second embodiment.

DETAILED DESCRIPTION OF EMBODIMENTS

The illustration in the drawings is schematically. In different drawings, similar or identical elements are provided with the same reference numerals.

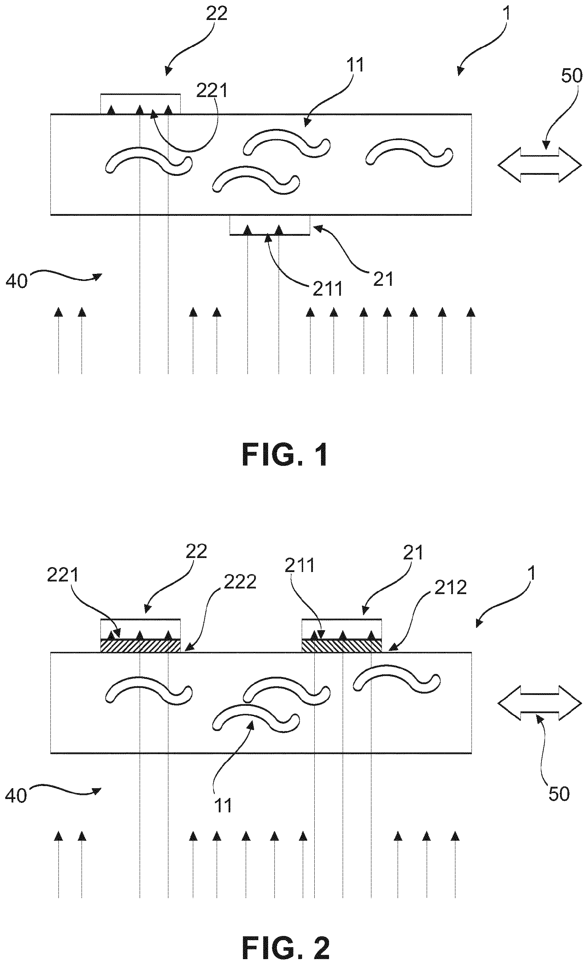

Fig. 1 represents schematically an arrangement of the first photo detector 21 and the second photo detector 22 on the user's skin 1 according to a first embodiment. In Fig. 1, the first photo detector 21 and the second photo detector 22 are arranged on opposite sides of the skin along a propagation direction of the ambient light 40 through the user's skin.

The first photo detector 21 is hereby arranged such that the ambient light 40 may be directly incident on it. In particular, the first sensor 211 of the first photo detector is arranged such as to face the source of the ambient light 40. The first signal obtained by the first photo detector 21 therefore comprises a first ambient light component indicative of the fluctuations in the ambient light 40 due to the user's movement 50 of the skin 1 as well as inherent fluctuations due to shades or changes in intensity, caused by environmental factors and/or by the user moving from one place to another.

The second photo detector 22 is arranged on the user's skin 1 such that the second sensor 221 of the second photo detector faces the user's skin. The second signal obtained by the second photo detector 22 comprises a second ambient light component indicative of the fluctuations inherent to the ambient light 40 and a modified ambient light component indicative of the alterations in the ambient light 40 that have been caused by encountering the blood vessels 11.

A processing unit (not shown) may use the first signal indicative of the fluctuations of the ambient light 40 to determine the alterations in the second signal that have been caused by said fluctuations. This allows the processing unit to extract a modified ambient light component from the first signal that does not comprise any alterations inherent to the ambient light 40. This modified ambient light component may then be used to determine one or more physiological parameters of the user.

Fig. 2 presents an arrangement of the first photo detector 21 and the second photo detector 22 according to a second embodiment. In this embodiment, the first photo detector 21 and the second photo detector 22 are arranged on the same side of the user's skin 1. Thus, the ambient light reaching both photo detectors has previously encountered the blood vessels 11 in the user's skin 1.

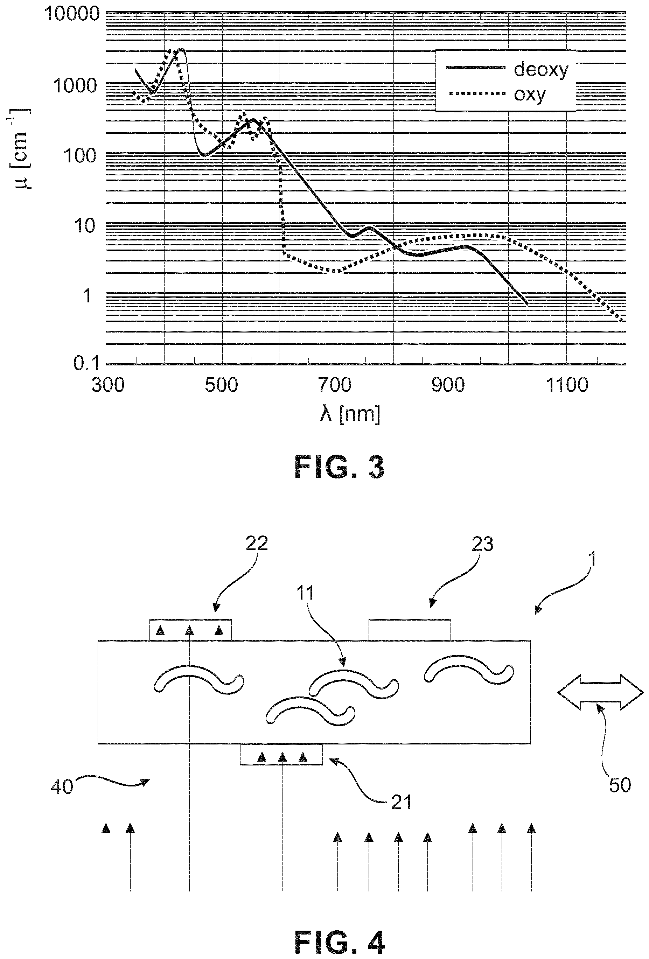

In order to use the first photo detector 21 to determine only the first ambient light component, a first, red filter 212 is placed in front of the first sensor 211 of the first photo detector 21. To that end, Fig. 3 illustrates the absorption $\mu$ of the blood vessels (per mg) as a function of the wavelength $\lambda$ (in [nm]). As may be appreciated from Fig. 3, the absorption of the blood vessels 11 in the red spectral regime is very small for both, oxygenated as well as deoxygenated blood. Accordingly, red spectral regime of the ambient light is only marginally influenced by encountering the blood vessels. Thus, in the red spectral regime, the modified ambient light component is negligible compared to the first ambient light component. Therefore, the first signal obtained by the first photo detector 21 may be considered as representing the first ambient light component without any modified light component.

In contrast to that, the second photo detector 22 is provided with a green filter 222 in front of the second sensor 221 in Fig. 2. As may again be appreciated from Fig. 3, the absorption of the ambient light by the blood vessels for both, oxygenated and deoxygenated blood, is very pronounced in the green spectral regime. Thus, by only letting the green spectral regime of the ambient light 40 pass to reach the second sensor 221, the second signal comprises a pronounced modified ambient light component. This is particularly so since the red spectral regime where the ambient light component would be stronger is cut off prior to reaching the second sensor 221. Subsequently, the first and second signal are processed by the processing unit at described herein above in relation to Fig. 1.

Fig. 4 schematically shows an arrangement of the first photo detector 21, the second photo detector 22 and an accelerometer 23 according to a modification of the first embodiment. The operation of the first photo detector 21 and the second photo detector 22 follows the scheme as described in relation to Fig. 1. Thus, in the modification according to Fig. 4, a first signal and a second signal as obtained by the first photo detector 21 and the second photo detector 22, respectively, are provided to the processing unit.

Additionally, an accelerometer 23 is employed to determine the motion of the user's skin. The accelerometer 23 then outputs a third signal indicative of said motion of the user's skin. The third signal is provided to the processing unit as well. The processing unit uses the third signal to determine which movement 50 of the skin has been performed during measurement of the first and second signal coming from the first photo detector 21 and the second photo detector 22. The processing unit uses this information to identify the artifacts and/or fluctuations in the ambient light that have been caused by movement 50 of the user's skin and uses this information to eliminate these artifacts prior to calculating the physiological parameters from the second signal.

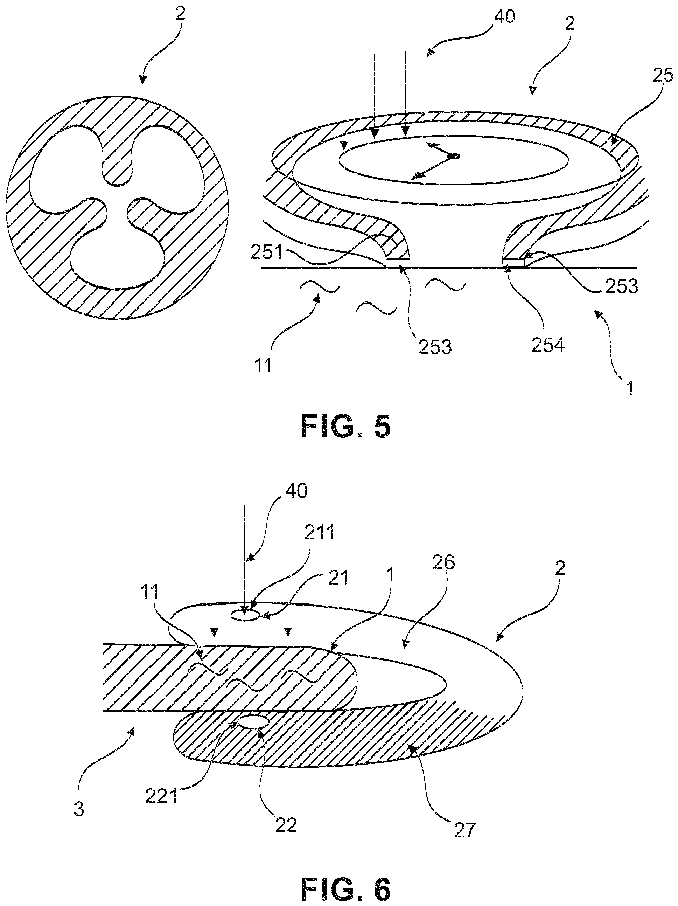

Fig. 5 represents a device 2 for determining a physiological parameter of a user using ambient light 40 according to a first embodiment including a light guide 25. In the present embodiment, the device 2 is a watch that can be worn around the wrist of a user. The ambient light 40 enters the light guide 25 from the top and the sides of the device 2. The ambient light 40 is then guided through the light guide 25 to the skin 1. The ambient light 40 exits the light guide 25 through a first outlet 251 provided with a first outlet filter 253 and a second outlet 252 with a second outlet filter 254. The first photo detector 21 and second photo detector 22 (not shown) are arranged adjacent to the first outlet 251 and the second outlet 252, respectively. The ambient light that has exited the light guide through the first outlet 251 and the second outlet 252 encounters the skin 1 and the blood vessels 11 therein and is reflected to the first photo detector 21 and the second photo detector 22. In this embodiment, the first photo detector 21 is provided with a red filter 212 and the second photo detector 22 is provided with a green filter 222 and the determination of the physiological parameter is performed as described in relation to Fig. 2.

Fig. 6 represents a device 2 for determining a physiological parameter of a user using ambient light 40 according to a second embodiment in which the device 2 is a finger clip that may be attached to the tip of a finger 3. The device 2 comprises a first attachment portion 26 and a second attachment portion 27.

The first attachment portion 26 is made from a transparent elastic material, such that the device may be clipped to the fingertip. The first photo detector 21 is attached to the outer surface of the first attachment portion 26 such that the sensor 211 of the first photo detector 21 faces the source of the ambient light 40. Thus, the first signal obtained by the first photo detector 21 comprises the first ambient light component.

The second attachment portion 27 is made from an opaque elastic material. Hereby, the second photo detector 22 is attached to the inner surface of the second attachment portion 27. Thus, the second photo detector 22 is facing the skin 1 of the finger 3. Accordingly, the second signal obtained by the second photo detector 22 comprises the second ambient light component and the modified ambient light component. Hereby, the first photo detector 21 and the second photo detector 22 are used to determine at least one physiological parameter using the first signal and the second signal as described in relation to Fig. 1.

Although in above described embodiments, the devices are worn around the wrist or attached to the ears or fingers, in other embodiments the devices may also be worn elsewhere, such as the upper arm, the ankle or the like.

Further, although in the above described embodiments the physiological parameters determined using the first and second photo detector measurements relate to the heart rate and the oxygen saturation of the blood, other physiological parameters may likewise be determined such as the blood pressure or the like.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the processing of the first and second signal or the determination of the physiological parameters et cetera performed by one or several units or devices can be performed by any other number of units or devices. These procedures performed by the improved portable device for determining at least one physiological parameter using ambient light and/or the processing unit, as well as the method performed to determine the physiological parameters can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a portable device for determining at least one physiological parameter of a user using ambient light. The device comprises a first photo detector adapted to obtain a first signal comprising a first ambient light component and a second photo detector adapted to obtain a second signal comprising a combination of a second ambient light component and a modified ambient light component. The device further comprises a processing unit adapted to determine the physiological parameter from the first signal and the second signal.

The device enables an improved determination of at least one physiological parameter using ambient light which has reduced power consumption.

## Claims

1. A portable device for determining at least one physiological parameter of a user using ambient light comprising:

   a first photo detector adapted to obtain a first signal comprising a first ambient light component;
   a second photo detector adapted to obtain a second signal comprising a combination of a second ambient light component and a modified ambient light component;
   a processing unit adapted to determine the physiological parameter from the first signal and the second signal.

2. The device according to claim 1, wherein
   the first photo detector is arranged on a first side of the user's skin;
   the second photo detector is arranged on a second side of the user's skin opposite the first side along a propagation direction of the ambient light through the user's skin.

3. The device according to claim 1, wherein
   the first photo detector and the second photo detector are arranged on a first side of the user's skin; and
   the first photo detector comprises a first filter adapted such that the first ambient light component may pass the first filter;
   the second photo detector comprises a second filter adapted such that the second ambient light component and the modified ambient light component may pass the second filter.

4. The device according to claim 3, wherein
   the first filter comprises a red and/or infrared filter; and
   the second filter comprises a green filter.

5. The device according to claim 3, further comprising an indicator, wherein
   the first photo detector comprises a first ultraviolet filter and the first signal obtained by the first photo detector comprises a first ultraviolet light component; and
   the second photo detector comprise a second ultraviolet filter and the second signal obtained by the second photo detector comprises a second ultraviolet light component; and wherein
   the processing unit is further adapted to provide a first indication to the indicator upon determining that a value of the first ultraviolet light component is above a pre-determined first ultraviolet light threshold and to provide a second indication to the indicator upon determining that a value of the second ultraviolet light component is above a pre-determined second ultraviolet light threshold.

6. The device according to claim 1, further comprising a light guide for guiding the ambient light to a user's skin, the light guide comprising a first outlet and a second outlet for directing the ambient light component to a first side of the user's skin.

7. The device according to claim 6, wherein the first photo detector is arranged adjacent to the first outlet and the second photo detector is arranged adjacent to the second outlet on the first side of the users skin.

8. The device according to claim 6, wherein the first outlet comprises a first filter and the second outlet comprises a second filter.

9. The device according to claim 1, further comprising an accelerometer arranged in close proximity to the second photo detector, wherein the accelerometer is adapted to output a third signal indicative of the motion of a user's skin; and the processing unit is adapted to determine the physiological parameter from the first signal, the second signal and the third signal.

10. The device according to claim 1, further comprising a light source and a controller unit; wherein the processing unit is further adapted to determine whether an intensity value of the ambient light is below or above a pre-defined threshold; and the processing unit is adapted to cause the controller unit to activate the light source when it is determined that the intensity value of the ambient light is below the pre-defined threshold.

11. The device according to claim 10, wherein the light source comprises one or more light emitting detectors; and the controller is further adapted to control the light emitting detectors to provide a change in intensity.

12. The device according to claim 1, further comprising a first attachment portion adapted for attaching the first photo detector, the first attachment portion comprising a transparent material; and a second attachment portion adapted for attaching the second photo detector, the second attachment portion comprising an opaque material.

13. The device according to claim 1, wherein the device is a wearable device, such as an ear clip, a finger clip or a wrist-worn device.

14. A method for determining a physiological parameter of a user using a portable device using ambient light comprising:

obtaining a first signal comprising a first ambient light component by a first photo detector;
obtaining a second signal comprising a combination of a second ambient light component and a modified ambient light component a second photo detector;
determining the physiological parameter from the first signal and the second signal using a processing unit.

**FIG. 1**

**FIG. 2**

EP 3 449 813 A1

FIG. 3

FIG. 4

13

**FIG. 5**

**FIG. 6**

Europäisches Patentamt
European Patent Office
Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/245637 A1 (MCKENNA EDWARD M [US]) 6 October 2011 (2011-10-06) * abstract; figures 1-11 * * paragraphs [0020] - [0058] * | 1-14 | INV. A61B5/00 A61B5/024 A61B5/1455 |
| X | US 2017/156650 A1 (BOWER CHRIS [GB] ET AL) 8 June 2017 (2017-06-08) * abstract; figures 1-5 * * paragraphs [0004] - [0027], [0035] - [0080] * | 1-14 | |
| X | US 2016/324432 A1 (AHMED WILLIAM [US] ET AL) 10 November 2016 (2016-11-10) | 1,2, 9-11,13, 14 | |
| A | * abstract; figures 5-8,27 * * paragraphs [0002] - [0006], [0009], [0054] - [0055], [0061] - [0063], [0069] - [0072] * * paragraphs [0077] - [0082], [0115], [0121] - [0123], [0127] - [0129], [0244] - [0253] * | 3-8,12 | |
| A | EP 3 033 992 A1 (NOKIA TECHNOLOGIES OY [FI]) 22 June 2016 (2016-06-22) * abstract; figures 1-5 * * paragraphs [0031] - [0054] * | 1-14 | TECHNICAL FIELDS SEARCHED (IPC) A61B |

The present search report has been drawn up for all claims

| Place of search | Date of completion of the search | Examiner |
|---|---|---|
| The Hague | 20 February 2018 | Carta, Riccardo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 449 813 A1**

## ANNEX TO THE EUROPEAN SEARCH REPORT
## ON EUROPEAN PATENT APPLICATION NO.

EP 17 18 9314

20-02-2018

| Patent document cited in search report | | Publication date | Patent family member(s) | | Publication date |
|---|---|---|---|---|---|
| US 2011245637 | A1 | 06-10-2011 | NONE | | |
| US 2017156650 | A1 | 08-06-2017 | CN | 106659406 A | 10-05-2017 |
| | | | GB | 2528055 A | 13-01-2016 |
| | | | US | 2017156650 A1 | 08-06-2017 |
| | | | WO | 2016005654 A1 | 14-01-2016 |
| US 2016324432 | A1 | 10-11-2016 | NONE | | |
| EP 3033992 | A1 | 22-06-2016 | EP | 3033992 A1 | 22-06-2016 |
| | | | JP | 2018502629 A | 01-02-2018 |
| | | | US | 2017354335 A1 | 14-12-2017 |
| | | | WO | 2016097472 A1 | 23-06-2016 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013038296 A